# EUROPÄISCHE PATENTSCHRIFT

(11) **EP 1 397 164 B1**
(45) Veröffentlichungstag und Bekanntmachung des Hinweises auf die Patenterteilung: **05.05.2010**
(21) Anmeldenummer: 02750947.0
(22) Anmeldetag: 18.05.2002
(51) Int. Cl.: A61L 2/07, A61L 2/06, A61L 2/18, A61L 2/20, A61L 2/26, A61M 5/00, A61M 5/178, A61M 5/32, B65D 51/00

(54) **VERSCHLUSSELEMENT**
SEALING ELEMENT
ELEMENT DE FERMETURE

(30) Priorität: 01.06.2001 DE 10127779
(43) Veröffentlichungstag der Anmeldung: 17.03.2004
(73) Patentinhaber: Arzneimittel GmbH Apotheker Vetter & Co. Ravensburg, 88212 Ravensburg (DE)
(72) Erfinder: VETTER, Udo, J., 88214 Ravensburg (DE); GLOCKER, Joachim, 88250 Weingarten (DE); SCHWARZ, Walter, 88339 Bad Waldsee (DE)
(74) Vertreter: Gleiss, Alf-Olav
(86) Internationale Anmeldenummer: PCT/EP2002/005505
(87) Internationale Veröffentlichungsnummer: WO 2002/098470

(56) Entgegenhaltungen:
- DE-A- 19 638 940
- GB-A- 847 913
- US-A- 3 986 508
- US-A- 5 741 236

## Beschreibung

Die Erfindung betrifft ein Verschlusselement gemäß Oberbegriff des Anspruchs 1.

Verschlusselemente und Verfahren der hier angesprochenen Art sind bekannt. Die Verschlusselemente dienen als Verschlussstopfen für sogenannte Primärpackmittel für medizinische Zwecke, beispielsweise für Spritzen und Karpulen. Es hat sich herausgestellt, dass Verschlusselemente der hier angesprochenen Art nicht in allen Fällen optimal sterilisiert werden können: Es gibt Bereiche, die bei einem Sterilisationsverfahren, beispielsweise beim Autoklavieren nicht unmittelbar von dem Sterilisationsmedium erreichbar sind, so dass eine einwandfreie Sterilisation nicht sichergestellt werden kann.

Die Schrift US-A-5 741 236 offenbart ein Verchlusselement gemäß dem Oberbegriff von Anspruch 1.

Es ist daher Aufgabe der Erfindung, ein Verschlusselement bereitzustellen, das sich dadurch auszeichnet, dass eine optimale Sterilisation möglich ist.

Zur Lösung dieser Aufgabe wird ein Verschlusselement vorgeschlagen, das die in Anspruch 1 genannten Merkmale umfasst. Es weist insbesondere mindestens zwei Teile auf, die zumindest bereichsweise aneinander anliegen, so dass Anlageflächen gebildet werden. Das Verschlusselement zeichnet sich dadurch aus, dass ein erstes Teil eine höhere Festigkeit als ein zweites Teil aufweist, dass das erste Teil als Stützkörper ausgebildet ist, durch den Kräfte aufnehmbar sind, die in das zweite Teil eingeleitet werden, und dass der Stützkörper als Sterilisationskörper ausgebildet ist, indem dampfdurchlässige Kanäle zur Sterilisation der Anlageflächen vorgesehen sind, die innerhalb des Sterilisationskörpers verlaufen.

Wesentlich ist, dass der Sterilisationskörper aufgrund der dampfdurchlässigen Kanäle für ein Sterilisationsmedium durchlässig ist, so dass er dieses Medium in Bereiche leitet, die sonst für die Sterilisation nicht zugänglich wären. Da die Kanäle innerhalb des Sterilisationskörpers verlaufen, ergibt sich für das Verschlusselement ein sehr einfacher Aufbau.

Weiterhin ist vorteilhaft, dass ein erstes Teil des Verschlusselements eine höhere Festigkeit aufweist als ein zweites, wobei das erste Teil als Stützkörper ausgebildet ist. Dieser erhöht die mechanische Festigkeit des Verschlusselements derart, dass dieses im Zusammenhang mit Applikationshilfen, beispielsweise mit motorisch angetriebenen Infusions- beziehungsweise Spritzenpumpen verwendet werden kann, ohne dass es aufgrund einer zu hohen Elastizität des Verschlusselements zu Störungen käme.

Ein weiteres bevorzugtes Ausführungsbeispiel zeichnet sich dadurch aus, dass der Sterilisationskörper als Klemmkörper ausgebildet ist. Damit ist es möglich, das Verschlusselement optimal zu sterilisieren und andererseits sicherzustellen, dass bei einfachem Aufbau eine Festklemmung des Verschlusselements beispielsweise an einem Spritzenkörper auf einfache Weise möglich ist.

Weitere Vorteile ergeben sich aus den übrigen Unteransprüchen.

Die Erfindung wird im Folgenden anhand der Zeichnung näher erläutert. Es zeigen:
- Figur 1: einen Schnitt eines ersten Ausführungsbeispiels eines zweiteiligen Verschlusselements;
- Figur 2: einen Schnitt eines Ausführungsbeispiels eines nicht zur Erfindung gehörigen vormontierten zweiteiligen Verschlusselements;
- Figur 3: eine Draufsicht auf die geöffnete Seite des in Figur 2 dargestellten Verschlusselements;
- Figur 4: einen Schnitt durch das in Figur 2 dargestellte Verschlusselement im montierten Zustand;
- Figur 5: einen Schnitt eines zweiten erfindungsgemäßen Ausführungsbeispiels mit außenliegendem Stützkörper;
- Figur 6: einen Schnitt eines dreiteiligen Verschlusselements mit einem Kupplungssystem und einer Vorrichtung zur Fluidführung;
- Figur 7: einen Schnitt eines weiteren Ausführungsbeispiels eines Verschlusselements mit einer Sicherheitskappe;
- Figur 8: einen Schnitt eines weiteren Ausführungsbeispiels eines Verschlusselements mit einer Filtereinrichtung;
- Figur 9 und 10: einen Schnitt durch weitere Ausführungsbeispiele des Verschlusselements.

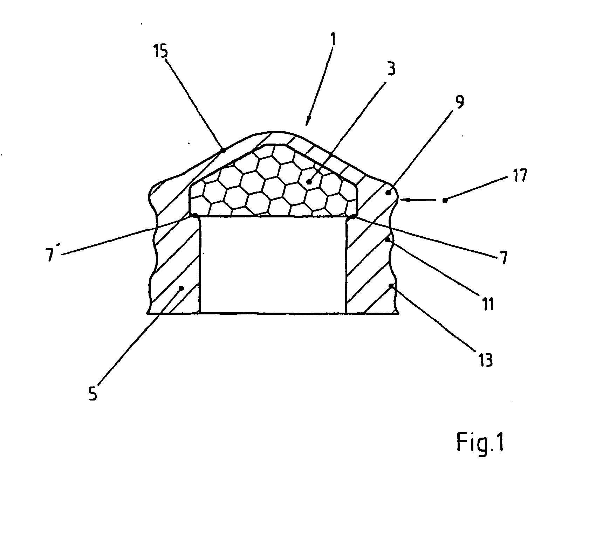

Figur 1 zeigt ein Verschlusselement 1, das aus einem formstabilen Stützkörper 3 und einer elastisch verformbaren Verschlusshülse 5 besteht. Der Stutzkörper 3 und die Verschlusshülse 5 sind als zylindrische Rotationskörper ausgeführt. Die Verschlusshülse 5 ist so ausgebildet, dass sie mit dem größten Teil der Oberfläche des Stützkörpers 3 in Anlagekontakt steht, diesen also zumindest teilweise umhüllt. Die Verschlusshülse 5 weist im Inneren eine umlaufende Stufe 7, 7' auf, die ebenfalls in Anlagekontakt mit dem Stützkörper 3 steht. Aufgrund der Elastizität der Verschlusshülse 5 und der Stufe 7, in die der Stützkörper 3 einrastbar ist, wird der Stützkörper 3 sicher gehalten.

Der Stützkörper 3 zeichnet sich dadurch aus, dass er eine höhere Festigkeit aufweist, als die Verschlusshülse 5. Er dient also dazu, das Verschlusselement 1 zu stabilisieren, insbesondere in dem Sinne, dass die Gesamtelastizität des Verschlusselements 1 reduziert wird, dieses also weniger kompressibel ist. Vorzugsweise ist der Stützkörper 3 so ausgebildet, dass er praktisch nicht komprimierbar ist.

Der Stützkörper 3 liegt mit Bereichen seiner Außenfläche an der Innenfläche der Verschlusshülse 5 an, so dass diese beiden Teile eine gemeinsame Anlagefläche bilden. Um bei einem Sterilisationsvorgang die Anlagefläche vollständig sterilisieren zu können, ist vorgesehen, dass der Stützkörper 3 durchlässige Kanäle aufweist, durch die ein Sterilisationsmedium zur Anlagefläche vordringen kann. Bei dem hier dargestellten Ausführungsbeispiel ist der Stützkörper 3 mit dampfpermeablen Strukturen versehen, die hier durch eine wabenförmige Schraffur gekennzeichnet sind. Es ist hier vorzugsweise vorgesehen, den gesamten Stützkörper mit einer derartigen dampfdurchlässigen Struktur zu versehen. Dadurch kann sichergestellt werden, dass bei Beaufschlagen des Verschlusselements 1 Dampf durch den gesamten Stützkörper 3 in den Bereich der Anlagefläche mit der Verschlusshülse 5 gelangen kann. Dies ermöglicht ein Sterilisieren aller Flächen, also auch der Flächen, mit denen der Stützkörper 3 und die Verschlusshülse 5 in Anlagekontakt stehen, in einem Arbeitsgang.

Das gezeigte Ausführungsbeispiel ist als Rotationskörper ausgeführt und wird bevorzugt zum Abdichten einer hier nicht dargestellten zylindrischen Röhre, nämlich eines Spritzenkörpers, eingesetzt. Die Außenseite der Verschlusshülse 5 weist umlaufende Wülste 9, 11, 13 auf. Zum Abdichten der zylindrischen Röhre wird das Verschlusselement 1 in diese eingesetzt. Damit treten die Wülste 9, 11, 13 in Anlagekontakt mit der Innenfläche der zylindrischen Röhre und bilden dabei drei umlaufende Dichtlippen, Das Verschlusselement 1 wird so in die zylindrische Röhre eingesetzt, dass dessen obere Fläche 15 gemeinsam mit der Innenwand der Röhre, also des Spritzenkörpers einen dicht abgeschlossenen Innenraum bildet, in dem ein Fluid, insbesondere ein flüssiges Medikament und/oder Nährlösung oder dergleichen dicht eingeschlossen werden kann. Es ist erkennbar, dass ein etwaiger Überdruck des Fluids, der auf die obere Fläche 15 der Verschlusshülse 5 wirkt, durch den dahinterliegenden Stützkörper 3 aufgenommen werden kann, Damit wird sicher eine ungewollte Verformung der Verschlusshülse 5 aufgrund der stützenden Wirkung des Stützkörpers 3 vermieden.

Bei einer Verlagerung des Verschlusselements 1 im Inneren der Röhre beziehungsweise eines Spritzenkörpers wird auf die der Fläche 15 der Verschlusshülse 5 abgewandte Seite des Stützkörpers 3 eine Kraft ausgeübt. Durch diese wird das Verschlusselement 1 in der Röhre - in Figur 1 nach oben - verlagert. Es wird deutlich, dass die Dicke der Wandung der Verschlusshülse 5 im Bereich der oberen Fläche 15 relativ gering ist, dass also von einem Kolben 5 auf den Stützkörper 3 ausgeübte Kräfte unmittelbar auf das Fluid ausgeübt werden, das in einem abgeschlossenen Raum der Spritze vorhanden ist. Bei einer Verlagerung des Verschlusselements 1 kann sich also aufgrund der geringen Wandstärke über dem Stützkörper 3 nur eine sehr geringe Verformung der Verschlusshülse 5 einstellen. Wird also ein Kolben zur Verlagerung des Verschlusselements 1 im Inneren eines Spritzenkörpers um einen gewissen Verlagerungsweg verschoben, so kann damit eine sehr exakt bestimmbare Flüssigkeitsmenge aus der Spritze ausgetragen werden. Dies gilt insbesondere dann, wenn die Elastizität des Stützkörpers 3 wesentlich geringer ist als die der Verschlusshülse 5.

An einem in eine zylindrische Röhre eingesetzten Verschlusselement 1, deren Innendurchmesser geringfügig kleiner ist als der Außendurchmesser des ersten Wulstes 9, entsteht zwischen dem ersten Wulst 9 und der Zylinderinnenwand eine dichtende Kraft, die hier durch einen Pfeil 17 angedeutet ist. Es ist zu erkennen, dass diese für die dichtende Wirkung erforderliche Kraft, ebenfalls durch den direkt dahinterliegenden Stützkörper 3 aufgenommen wird. Das heißt sowohl die dichtende Kraft, wie auch die durch etwaigen Überdruck des abzudichtenden Fluids entstehenden Kräfte werden durch den Stützkörper 3 aufgenommen. Die Wülste 11, 13 sind nicht mit so hohen Dichtkräften beaufschlagt wie der erste Wulst 9; sie dienen dem Auffangen einer etwaigen Leckage, die an dem ersten Wulst 9 entstehen, könnte. Der Stützkörper 3 ist damit so auf der Innenseite der Verschlusshülse 5 angeordnet, dass alle wesentlichen Kräfte, die auf die Verschlusshülse 5 einwirken, sicher vom Stützkörper 3 aufgenommen werden können. Die Versählusshülse 5 kann aus einem für die gewünschte Abdichtung des Innenraums des Spritzenkörpers optimalen, auch relativ weichen Material bestehen. Sie verhält sich jedoch im Zusammenwirken mit dem stabilen Stützkörper 3 wie ein wesentlich steiferes Bauteil und ist damit wirkungsvoll gegen ungewollte Verformungen durch auf die Verschlusshülse 5 wirkende Kräfte geschützt.

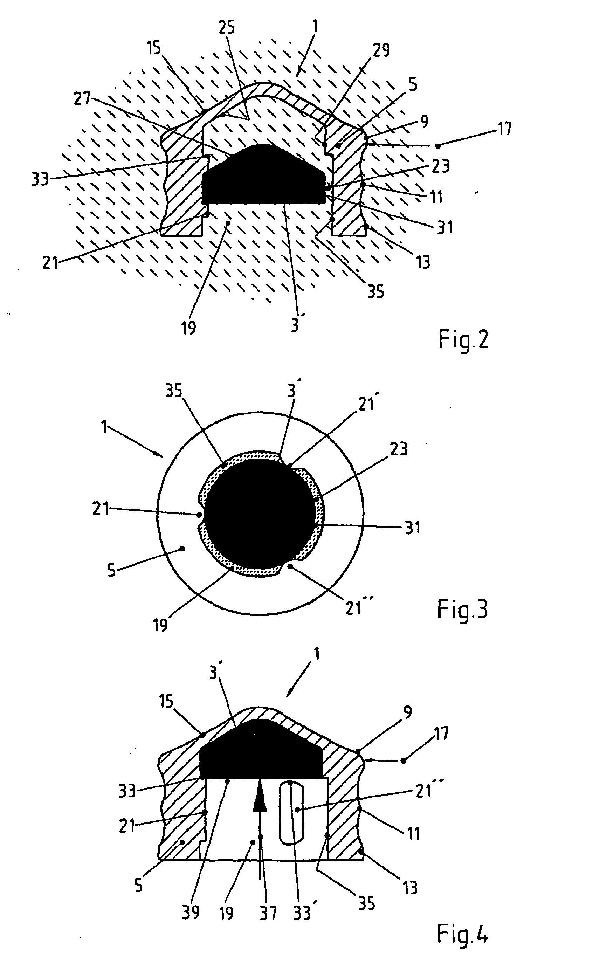

Figur 2 zeigt ein nicht zur Erfindung gehöriges, ebenfalls als Rotationskörper ausgeführtes Ausführungsbeispiel eines Verschlusselements. Gleiche Teile sind mit gleichen Bezugsziffern versehen, so dass auf die Beschreibung zu Figur 1 verwiesen wird.

Der wesentliche Unterschied zur vorhergehenden Ausführungsfomn liegt darin, dass der Stützkörper 3' keine dampfpermeablen Strukturen aufweist. Der 'Stützkörper 3' ist in einer ersten Vormontageposition dargestellt. Die Verschlusshülse 5 weist eine im Wesentlichen zylindrische Ausnehmung 19 auf, deren Durchmesser größer ist als der Außendurchmesser des Stützkörpers 3'. Die Innenfläche 35 der Ausnehmung 19 weist mindestens zwei vorzugsweise drei Rippen auf, von denen hier im Schnitt die Rippe 21 sichtbar ist. Die Rippen schließen einen gedachten Innenkreis ein, dessen Innendurchmesser etwa dem Außendurchmesser des Stützkörpers 3' entspricht. Damit können die Rippen eine Haltevorrichtung bilden: Sie liegen an der Außenfläche des Stützkörpers 3' so mit einer gewissen Vorspannung an, dass dieser sicher in der ersten Vormontageposition gehalten wird. Die Rippen halten den Stützkörper 3' in einem Abstand zur Innenfläche 35 der Verschlusshülse 5. Da also die Ausnehmung 19 im Inneren der Verschlusshülse 5 einen Innendurchmesser aufweist, der größer ist als der Außendurchmesser des Stützkörpers 3 werden in Richtung der Rippen 21 verlaufende Spalte 23 gebildet, die in Längsrichtung von den Rippen voneinander getrennt sind. Diese Spalten bilden damit Kanäle aus, durch die ein Sterilisationsmedium an den Seiten des Stützkörpers 3' vorbei in den Raum innerhalb der Verschlusshülse 5 gelangen kann, der oberhalb des Stützkörpers 3' angeordnet ist. Damit kann die gesamte Ausnehmung 19 der Verschlusshülse 5 und die komplette Außenfläche des Stützkörpers 3' sterilisiert werden. Wird also beispielsweise heißer Dampf zur Sterilisation eingesetzt, der hier durch eine Schraffur angedeutet ist, können alle innen- und außenliegenden Oberflächen der Verschlusshülse 5 und alle Oberflächen des Stützkörpers 3' erreicht und damit sterilisiert werden.

In Figur 2 ist auch erkennbar, dass die Ausnehmung 19 oben durch eine Anlagefläche 25 begrenzt wird, deren Kontur an die obere Kontur und damit Anlagefläche 27 des Stützkörpers 3' angepasst ist. Im oberen Bereich der Ausnehmung 19 findet sich eine umlaufende Anlagefläche 29, die mit einer umlaufenden Anlagefläche 31 des Stützkörpers 3' zusammenwirkt, wenn dieser aus der in Figur 2 wiedergegebenen Vormontageposition in seine spätere EndmontagePosition verlagert wird, auf die unten noch näher eingegangen wird. Des Weiteren ist in Figur 2 zu erkennen, dass das obere Ende 33 der Rippe 21, die hier im Schnitt dargestellt ist, eine Rastvorrichtung bildet, die den Stützkörper 3' verriegelnd hält, wenn dieser in seine Endmontageposition verlagert wird.

Figur 3 zeigt eine Unteransicht in die Ausnehmung 19, des in Figur 2 dargestellten, nicht zur Erfindung gehörenden Verschlusselements 1. Zu erkennen ist die Verschlusshülse 5 und die Ausnehmung 19 mit den Rippen 21, 21', 21". Diese stehen linienförmig in Anlagekontakt mit dem Stützkörper 3', der dadurch sicher in der ersten Vormontageposition gehalten wird. Weiterhin zu erkennen ist der lediglich durch die Rippen 21, 21', 21" unterbrochene umlaufende Spalt 23, der hier gepunktet dargestellt ist. Durch den Spalt 23, der durch die umlaufende Anlagekontaktfläche 31 des Stützkörpers 3' und der Innenfläche 35 der Verschlusshülse 5 gebildet wird, kann zur Sterilisation vorgesehener heißer Dampf hindurchströmen. Somit ist gewährleistet, dass das gesamte Verschlusseliement 1 im vormontierten Zustand, in einem Arbeitsgang autoklaviert, also sterilisiert werden kann. Die für einen sicheren Halt des Stützkörpers 3' erforderlichen Haltekräfte an den Rippen 21, 21', 21", werden dabei durch die Eigenelastizität der Verschlusshülse 5' aufgebracht.

Figur 4 zeigt nochmals dieselbe Ansicht wie Figur 2 mit dem Unterschied, dass der Stützkörper 3' sich in einer zweiten Endmontageposition befindet. Gleiche Teile sind mit gleichen Bezugsziffern versehen, so dass auf die Beschreibung zu den vorhergehenden Figuren verwiesen wird. Zu erkennen ist, dass die in Figur 2 erläuterten Anlageflächen 25 bis 31 nun in Anlagekontakt stehen. Durch den Pfeil 37 wird angedeutet, dass der Stützkörper 3' mittels einer geeigneten Vorrichtung so lange in Richtung des Pfeiles 37 verschoben wird, bis dieser in der Endposition einrastet. Zu erkennen ist auch, wie der Stützkörper 3' an den oberen Enden 33 und 33' der Rippen 21, 21', 21" verrastet. Der Stützkörper 3' wird dadurch sicher durch die Rastkräfte, Adhäsionskräfte sowie Reibkräfte an den Anlagekontaktflächen 25 bis 31 gehalten. Die für die Reibung erforderlichen Kräfte werden insbesondere durch die Eigenelastizität der Verschlusshülse 5 aufgebracht.

Das Verschlusselement 1 kann im montierten und eingesetzten Zustand entlang des Pfeiles 37 in einem Spritzenkörper auf bekannte Weise mittels eines Kolbens verschoben werden, der auf den Stützkörper 3' in Richtung des Pfeils 37 wirkende Kräfte ausübt. Dadurch kann, ein sich in einer zylindrischen Röhre befindliches Fluid, unter Überdruck gesetzt werden, was zum dosierten Entleeren der zylindrischen Röhre ausgenutzt werden kann. Insbesondere handelt es sich hierbei um Spritzen oder Karpulen zur Injektion von flüssigen Medikamenten und/oder Nährlösungen oder dergleichen. Dabei ist eine feine Dosierbarkeit der Injektionsmenge, vor allem in automatisierten Injektionsvorrichtungen, besonders wichtig. Eine feine Dosierung erfolgt dabei durch ein gezieltes Verschieben des Verschlusselements 1 in Richtung des Pfeiles 37. Die zum Verschieben des Verschlusselements 1 notwendige Kraft greift dabei vorteilhafterweise an der unteren Fläche 39 des Stützkörpers 3' in Richtung des Pfeiles 37 an. Diese Kraft wird direkt über den Stützkörper 3' über die oberen Anlagekontaktflächen 25 und 27 auf die Verschlusshülse 5 übertragen. Auch hieraus wird deutlich, dass aufgrund der großflächigen Kraftübertragung und der formgebenden Wirkung des Stützkörpers 3', eine ungewollte Verformung der Verschlusshülse 5 praktisch ausgeschlossen ist. Das heißt, durch eine Verschiebung des Verschlusselements 1 mittels einer Kraft in Richtung des Pfeils 37 lässt sich eine zylindrische Röhre, die über eine zweite Öffnung verfügt, insbesondere eine Spritze oder Karpule, fein dosiert, insbesondere zur medizinischen Injektion von flüssigen Medikamenten und/oder Nährlösungen oder dergleichen, entleeren.

In Figur 4 ist zu erkennen, dass etwaige Kräfte eines in einer Röhre beziehungsweise in einem Spritzenkörper eingeschlossenen Fluids auf die obere Fläche 15 der Verschlusshülse 5 sowie auftretende Dichtkräfte, die durch den Pfeil 17 angedeutet sind, durch den im Inneren des Verschlusselements 1 angeordneten Stützkörper 3' aufgefangen werden.

Die hier dargestellten Ausführungsformen des Verschlusselements 1 als Rotationskörper sind keinesfalls bindend. So lässt sich das Prinzip einer elastischen Verschlusshülse mit einem Stützkörper in jeder Art von Röhre beziehungsweise Spritzenkörper realisieren, wie zum Beispiel in ovalen, mehreckigen Röhren realisieren, auch in Röhren mit frei gewählten Querschnittsflächen.

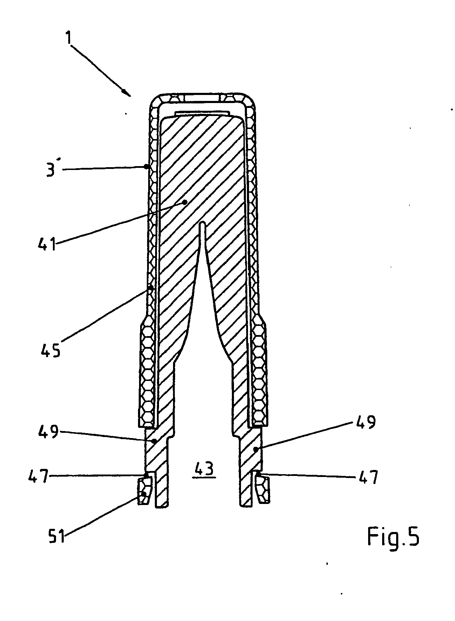

Figur 5 zeigt ein zweites erfindungsgemäßes Ausführungsbeispiel eines Verschlusselements 1, das zwei Teile umfasst, nämlich eine Verschlusskappe 41, die in ihrem in Figur 5 unteren Bereich hohl ausgebildet ist und einen Innenraum 43 umfasst, in den die Spitze einer Spritze gemeinsam mit einer Injektionsnadel einführbar ist. Diese kann in den Grundkörper der Verschlusskappe 41 eingestochen werden, wenn das Verschlusselement 1 auf die hier nicht dargestellte 10 Spritze aufgesetzt wird.

Die verschlusskappe 41 wird von einer Sicherheitskappe 45 umgeben, die über die Verschlusskappe 41 stülpbar ist. In dem unteren Bereich der Verschlusskappe 41, also im Bereich des Innenraums 43 15 ist die Sicherheitskappe 45 mit Ausnehmungen 47 versehen, in die Vorsprünge 49 der Verschlusskappe 41 hineinragen. Die Ausnehmungen 47 dienen damit als Sichtfenster.

Die Sicherheitskappe 45 setzt sich nach unten in einen Ring 51 fort, der den unteren Rand der Verschlusskappe 41 umgibt und die Vorsprünge 49 so untergreift, dass bei der Ausübung von Zugkräften auf die Sicherheitskappe 45 die Verschlusskappe 41 des Verschlusselements 1 von einer Spritze abgezogen wird.

Die Sicherheitskappe 45 stabilisiert die Verschlusskappe 41 und dient damit als Stützkörper 3' des Verschlusselements 1. Die Verschlusskappe 41 füllt den Innenraum der Sicherheitskappe 45 praktisch ganz aus. Es ergeben sich damit Anlageflächen zwischen der Innenfläche der Sicherheitskappe 45 und der Außenfläche der Verschlusskappe 41. Um sicherzugehen, dass im Bereich der Anlagefläche vorhandene Verunreinigungen inaktivierbar sind, ist die Sicherheitskappe 45 zumindest mit Kanälen versehen, die die Wandung der Sicherheitskappe 45 durchdringen und bis zum Bereich der Anlagefläche führen. Vorzugsweise ist die Sicherheitskappe 45 gänzlich aus einem porösen Material hergestellt, das dem des Stützkörpers entspricht, der anhand der Figuren 1 bis 4 erläutert wurde. Beispielsweise kann hier wie dort ein sinterähnliches Material zur Herstellung des Stützkörpers eingesetzt werden. Wird das Verschlusselement 1, das in Figur 5 dargestellt ist, in ein Sterilisationsmedium eingebracht, beispielsweise unter Heißdampf gesetzt, so kann dieses Medium die Außenfläche der Sicherheitskappe 45, den Innenraum 43 der Verschlusskappe 41, aber eben auch die Anlagefläche zwischen den beiden Teilen sicher erreichen, weil das Material der Sicherheitskappe 45 dampfpermeabel ist, also durchlässig für das Sterilisationsmedium. Denkbar ist es auch, die Verschlusskappe 41 mit Kanälen, porösen Strukturen oder gänzlich aus porösem Material herzustellen, um im Anlagebereich beziehungsweise im Bereich der Anlagefläche eine Sterilisation durchführen zu können. Bei dem in Figur 5 dargestellten Ausführungsbeispiel können aber auch Kanäle in den Bereich der Anlagefläche führen, wie sie anhand der Figuren 2 und 3 erläutert wurden, um eine Sterilisation durchführen zu können.

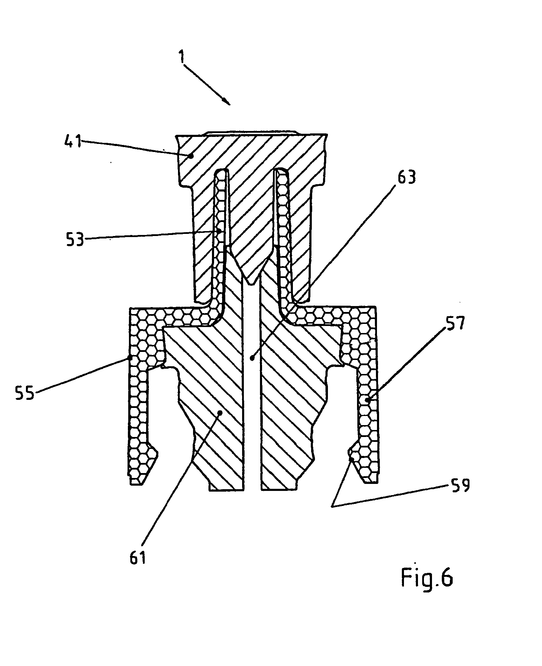

Die Schnittdarstellung gemäß Figur 6 zeigt ein weiteres Ausführungsbeispiel eines Verschlusselements 1, das eine Verschlusskappe 41 umfasst. Diese ist auf einen konischen Ansatz 53 eines Klemmkörpers 55 aufgesetzt, auf den gegebenenfalls auch eine Injektionsnadel aufsetzbar ist. Der Klemmkörper 55 weist einen zylindrischen Mantel 57 auf, der den Endbereich eines zylindrischen Rohres beziehungsweise eines Spritzenkörpers übergreift, der hier allerdings nicht dargestellt ist. Im der Verschlusskappe 41 abgewandten unteren Endbereich des Mantels 57 sind auf dessen Innenseite als Widerhaken 59 dienende Vorsprünge vorgesehen, die außen an dem Rohr beziehungsweise Spritzenkörper verriegelt angreifen, um den Klemmkörper 55 sicher am Rohr beziehungsweise Spritzenkörper zu verankern. Im Inneren des Klemmkörpers 55 ist ein Verschlussstopfen 61 vorgesehen, der den Endbereich des Rohrs beziehungsweise des Spritzenkörpers dichtend abschließt und bis in den konischen Ansatz 53 hineinragt. Der Verschlussstopfen 61 weist einen zentralen Durchgangskanal 63 auf, der von dem Verschlussstopfen 61 dicht abgeschlossen wird.

Die Schnittdarstellung gemäß Figur 6 lässt erkennen, dass die Teile des Verschlusselements 1, hier also die Verschlusskappe 41 und der Verschlussstopfen 61 nicht unmittelbar aufeinandergreifen, also nicht direkt aneinander anliegen. In dem Anlagebereich zwischen den beiden Teilen befindet sich der Klemmkörper 55, der Kanäle oder poröse Strukturen aufweisen kann. Hier ist vorgesehen, dass der gesamte Klemmkörper 55 aus einem porösen, gegebenenfalls sinterähnlichen Material besteht. Damit ist es möglich, in einem Autoklavier- beziehungsweise Sterilisationsverfahren den sonst unzugänglichen Bereich zwischen den Teilen des Verschlusselements 1 zu erreichen und Verunreinigungen zu inaktivieren. Der Klemmkörper 55 wirkt damit als Sterilisationskörper. Er ermöglicht den Zutritt des Sterilisationsmediums in den Bereich, in dem die Verschlusskappe 41 am Klemmkörper 55 und dieser am Verschlussstopfen 61 anliegt. Da das Sterilisationsmedium von außen und von innen an das Verschlusselement 1 herantreten kann ist dieses vollständig sterilisierbar.

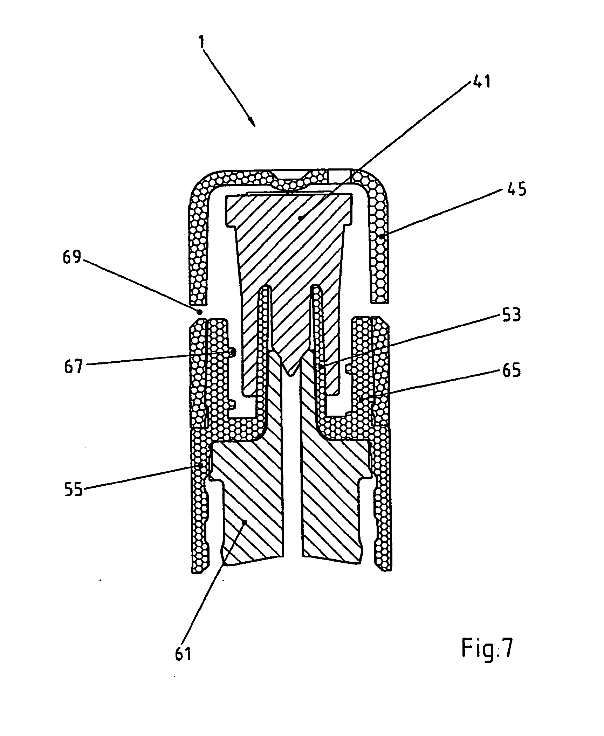

Das in Figur 7 dargestellte Ausführungsbeispiel eines Verschlusselements 1 ist grundsätzlich so aufgebaut, wie das in Figur 6 dargestellte: Es umfasst eine Verschlusskappe 41, einen als Sterilisationskörper dienenden Klemmkörper 55 und einen Verschlussstopfen 61, der, wie bei dem Ausführungsbeispiel gemäß Figur 6, im Inneren des Klemmkörpers 55 angeordnet ist und dazu dient, ein Rohr beziehungsweise einen Spritzenkörper auf den der Klemmkörper 55 aufgesetzt wird, im Endbereich dichtend zu erschließen. Durch den zwischen Verschlusskappe 41 und Verschlussstopfen 61 liegenden Klemmkörper 55, der hier beispielhaft vollständig aus porösem Material besteht, ist wiederum eine Sterilisation des von den beiden Verschlusselementen eingeschlossenen Bereichs möglich. Der Klemmkörper 55 weist hier einen den konischen Ansatz 53 umgebenden zylindrischen Ansatz 65 auf, der auf seiner Innenseite mit einem Innengewinde 67 versehen sein kann. Dieses dient dazu, eine auf den konischen Ansatz 53 aufgebrachte Kanüle sicher zu halten und am Klemmkörper 55 zu verankern. Zusätzlich ist hier noch eine Sicherheitskappe 45 vorgesehen, die die Verschlusskappe 41 übergreift und sicherstellt, dass diese nicht beschädigt oder versehentlich aus dem Verbund des Verschlusselements 1 entfernt wird. Die Sicherheitskappe 45 umgreift den zylindrischen Ansatz 65 und ist an diesem verriegelnd verankert. Durch eine Lücke ist eine Sollbruchstelle 69 angedeutet: Wird eine Kraft auf die Sicherheitskappe 45 ausgeübt, so wird der obere Teil derselben abgebrochen, so dass die Verschlusskappe 41 zugänglich und abnehmbar wird. Ein Verbraucher kann also ohne weiteres sehen, ob am Verschlusselement 1 irgendwelche unerlaubten Manipulationen durchgeführt wurden. Die Sicherheitskappe 45 stellt damit einen sogenannten Originalitätsverschluss oder Garantieverschluss dar.

Bei dem hier dargestellten Ausführungsbeispiel ist die Sicherheitskappe 45 mit Kanälen oder mit porösen Strukturen versehen, um die Außenfläche der 20 Verschlusskappe 41 in einem Sterilisationsverfahren erreichen zu können. Denkbar ist es auch, wie in Figur 7 dargestellt, die komplette Sicherheitskappe 45 aus einem porösen Material herzustellen.

Aus den Erläuterung zu Figur 7 wird deutlich, dass bei dem hier dargestellten Ausführungsbeispiel, wie bei dem Ausführungsbeispiel gemäß Figur 6, der Klemmkörper 55 als Sterilisationskörper dient, aber auch dazu, das Verschlusselement 1 in sich zu stabilisieren. Damit wird erreicht, dass der Klemmkörper 55 letztlich auch als. Stützkörper des Verschlusselements 1 dient.

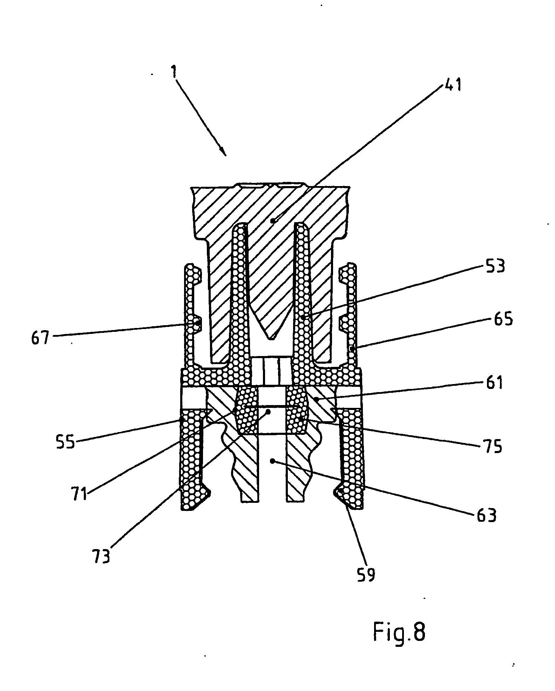

Figur 8 zeigt ein weiteres Ausführungsbeispiel eines Verschlusselements 1, das im Wesentlichen dem entspricht, das anhand von Figur 7 erläutert wurde. Gleiche Teile sind daher mit gleichen Bezugsziffern versehen. Allerdings ist bei der Darstellung gemäß Figur 8 die Sicherheitskappe 45 nicht aufgesetzt. Unterschiedlich gegenüber den bisher beschriebenen Ausführungsbeispielen ist, dass im Bereich des Durchgangskanals 63 des Verschlussstopfens 61 eine Filtereinrichtung 71 vorgesehen ist, die einen im Strömungspfad des aus dem Rohr beziehungsweise Spritzenkörpers austretenden Mediums einen Filter 73 aufweist, der von einer Halterung 75 fixiert wird. Die Halterung 75 wird hier durch einen Ring gebildet, der seinerseits aus porösem Material besteht, so dass seine Anlageflächen am Verschlussstopfen 61 sterilisierbar sind. Der Ring der Halterung 75 ist hier als separates Element dargestellt. Es ist aber auch möglich, dass die Halterung 75 Teil des Klemmkörpers 55 ist. Der Ring ragt so weit in den Verschlussstopfen 61 ein, dass er seinerseits als Stützkörper für den Verschlussstopfen 61 dient und sicherstellt, dass dieser dichtend an ein Rohr beziehungsweise einen Spritzenkörper angepresst wird, wenn das Verschlusselement 1 aufgesetzt wird.

Rein beispielhaft ist bei dem in Figur 8 dargestellten Ausführungsbeispiel wiederum ein zylindrischer Ansatz 65 vorgesehen, der den konischen Ansatz 53 umgibt und mit einem Innengewinde 67 versehen ist.

Figur 8 zeigt auch, dass der als Sterilisationskörper dienende Klemmkörper 55 so in das Innere der Verschlusskappe 41 greift, dass diese stabilisiert wird. Der Klemmkörper wirkt hier also ebenfalls als Stützkörper.

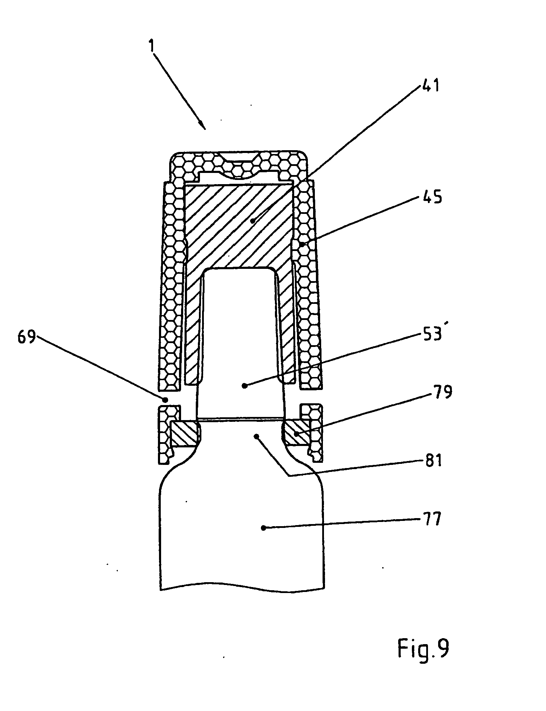

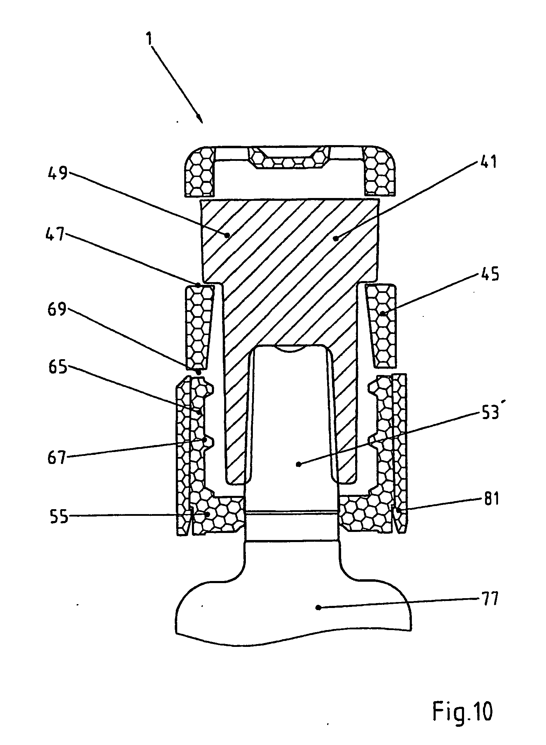

In den Figuren 9 und 10 sind nochmals Ausführungsbeispiele von Verschlusselementen dargestellt, hier allerdings mit dem oberen Teil eines Spritzenkörpers 77, der einen konischen Ansatz 53' aufweist, auf den eine Kanüle aufsteckbar ist. Bei den in Figur 9 dargestellten Ausführungsbeispielen ist das Verschlusselement 1 ähnlich ausgebildet wie das anhand von Figur 5 dargestellte Ausführungsbeispiel: Das Verschlusselement 1 weist eine Verschlusskappe 41 auf, die von einer Sicherheitskappe 45 übergriffen wird. Die Höhe der Sicherheitskappe 45 ist so gewählt, dass sie so weit nach unten über die Verschlusskappe 41 reicht, dass am unteren Rand der Sicherheitskappe 45 ein Haltering 79 vorgesehen werden kann, der in einer entsprechenden Vertiefung 81, die auf der Außenfläche des Spritzenkörpers 77 umläuft, eingreift und damit die Sicherheitskappe 45 am Spritzenkörper 77 verankert. Durch eine Lücke ist in Figur 9 eine umlaufende Sollbruchstelle 69 angedeutet. Wird eine Kraft auf die Sicherheitskappe 45 ausgeübt, so reißt deren oberer Teil mit der Verschlusskappe 41 ab. Der untere Rand verbleibt mit dem Haltering 79 am Spritzenkörper 77.

Durch die Sollbruchstelle 69 kann ein Sterilisationsmedium in das Innere der Sicherheitskappe 45 gelangen und Bereiche sowohl der Außenfläche des konischen Ansatzes 53' als auch des Halterings 79 sterilisieren. Hier ist vorgesehen, dass die Sicherheitskappe 45 gänzlich aus porösem Material hergestellt ist, um die Anlagefläche zwischen Verschlusskappe 41 und Sicherheitskappe 45 für das Sterilisationsmedium zugänglich zu machen. Es würde grundsätzlich jedoch ausreichen, hier Kanäle oder poröse Strukturen vorzusehen, um die Anlagefläche zugänglich zu machen. Schließlich ist es auch hier denkbar, im Anlagebereich zwischen den beiden Teilen des Verschlusselements 1 Vorsprünge und Vertiefungen zur Ausbildung von Kanälen vorzusehen, um eine Sterilisation des Anlagebereichs zu ermöglichen. Dies wurde anhand der Figuren 2 bis 4 für ein anderes Ausführungsbeispiel bereits erläutert. Dieses Grundprinzip kann hier jedoch ohne weiteres auf das in Figur 9 dargestellte Ausführungsbeispiel übertragen werden.

Aus der Darstellung gemäß Figur 9 ist wiederum erkennbar, dass die Sicherheitskappe 45 auch der Stabilisierung der Verschlusskappe 41 dient und diese dichtend an die Außenfläche des konischen Ansatzes 53' anpresst. Damit zeigt die Sicherheitskappe 45 auch die Funktion eines Stützkörpers.

Die Schnittdarstellung gemäß Figur 10 zeigt ein abgewandeltes Ausführungsbeispiel eines Verschlusselements 1. Es weist eine Verschlusskappe 41 auf, die, wie bereits beschrieben, auf den konischen Ansatz 53' eines Spritzenkörpers 77 aufsetzbar ist. Das Verschlusselement umfasst noch einen Klemmkörper 55, der außen am Spritzenkörper 77 beziehungsweise im unteren Bereich des konischen Ansatzes 53' angreift und das Verschlusselement 1 hält. Der Klemmkörper 55 weist einen zylindrischen Ansatz 65 mit einem Innengewinde 67 auf, das dazu dient, eine auf den konischen Ansatz 53' aufgesetzte Kanüle sicher zu halten.

Außen auf den Klemmkörper 55 ist eine Sicherheitskappe 45 aufgesetzt, die durch eine Nase 81 verriegelnd auf der Außenseite des Klemmkörpers 55 eingreift und die Verschlusskappe 41 überspannt. In die Mantelfläche der Sicherheitskappe 45 sind Ausnehmungen 47 eingebracht, in die Vorsprünge 49 der Verschlusskappe 41 eingreifen. Die Ausnehmungen 47 dienen einerseits als Sichtfenster, andererseits bieten Sie Anlageschultern für die Vorsprünge 49, um die Verschlusskappe 41 gemeinsam mit der Sicherheitskappe 45 von dem Spritzenkörper 77 abnehmen zu können. Durch eine Lücke ist auch hier eine umlaufende Sollbruchstelle 69 angedeutet, die der Realisierung eines Originalitätsverschlusses dient und dazu führt, dass der obere Teil der Sicherheitskappe 45 abbricht, wenn entsprechend große Kräfte ausgeübt werden. Mit dem abgebrochenen Teil der Sicherheitskappe 45 wird dann die Verschlusskappe 41 des Verschlusselements 1 von dem Spritzenkörper 77 abgenommen, so dass der konische Ansatz 53' frei zugänglich ist.

Bei dem hier dargestellten Ausführungsbeispiel sind sowohl der Klemmkörper 55 als auch die Sicherheitskappe 45 aus porösem Material hergestellt, damit ist sichergestellt, dass die Anlageflächen der Teile des Verschlusselements 1 in einem Sterilisationsverfahren sicher erreichbar sind: Einerseits wird der Berührungsbereich zwischen Sicherheitskappe 45 und Klemmkörper 55 sicher sterilisiert, andererseits alle Anlageflächen zwischen der Sicherheitskappe 45 und der Verschlusskappe 41.

Die Sicherheitskappe 45 dient auch dazu, die Verschlusskappe 41 zu stabilisieren. Damit ist auch hier ein Stützkörper für das Verschlusselement 1 realisiert.

Aus den Erläuterungen zu den Figuren 1 bis 10 wird Folgendes deutlich:

Das Verschlusselement ist so ausgebildet, dass Berührungsbereiche beziehungsweise Anlageflächen zwischen den einzelnen Teilen des Verschlusselements in einem Sterilisationsverfahren zugänglich sind. Dies ist dadurch möglich, dass ein ganzes Teil mit Kanälen, porösen Strukturen oder dergleichen versehen wird beziehungsweise vollständig aus einem porösen Material hergestellt ist. Dies wurde anhand des Stützkörpers näher erläutert, der in Figur 1 dargestellt ist.

Das Verschlusselement zeichnet sich also dadurch aus, dass Anlageflächen unmittelbar von Sterilisationsmedien erreichbar sind. Denkbar ist es auch, dass zwei Teile, hier beispielsweise eine Verschlusskappe 41 und ein Verschlussstopfen 61 nicht unmittelbar aufeinander anliegen, sondern an einem dazwischen liegenden Sterilisationskörper. Bei den hier dargestellten Ausführungsbeispielen war der Sterilisationskörper gleichzeitig als Klemmkörper 55 ausgebildet. Denkbar ist es, dass hier auch eine Stabilisierungsfunktion übernommen wird.

Wird bei einem Verschlusselement ein Stützkörper vorgesehen, wie er anhand der Figuren 1 bis 4 erläutert wurde, so kann das Verschlusselement 1 insbesondere im Zusammenhang mit Spritzensystemen eingesetzt werden, bei denen das Verschlusselement mit einem geeigneten Antrieb versehen und über einen Kolben mit Druckkräften beaufschlagt wird. Die automatischen Systeme sind in der Regel so ausgebildet, dass bei irgendwelchen Unregelmäßigkeiten, die insbesondere auf einer unerwünschten Elastizität des Verschlusselements beruhen, eine automatische Abschaltung erfolgt Durch die Stabilisierung des Verschlusselements durch den Stützkörper können derartige Fehlfunktionen mit hoher Sicherheit ausgeschlossen werden.

Es hat sich im Übrigen gezeigt, dass bei Verwendung eines Sterilisationskörpers dieser verschiedene Funktionen übernehmen kann. Er kann als Klemmkörper zur Fixierung des Verschlusselements an einem Spritzenkörper dienen aber auch dazu, eine Kanüle sicher an einem konischen Ansatz des Klemmkörpers oder an dem konischen Ansatz eines Spritzenkörpers zu halten.

In allen Fällen ist das Verschlusselement einfach aufgebaut und dadurch in weiten Bereichen der Medizin sicher einsetzbar, dass eine vollständige Sterilisierung der gegebenen Anlageflächen von Teilen des Verschlusselements gewährleistet ist.

Der anhand der Figuren 1 bis 4 erläuterte Stützkörper zeichnet sich durch eine wesentlich höhere Steifigkeit aus, als sie bei der Verschlusshülse gegeben ist. Aber auch bei den anderen hier erläuterten Ausführungsbeispielen ist bei dem Verschlusselement mit Hilfe des Sterilisationskörpers der als Klemmkörper und/oder als Sicherheitskappe ausgebildet sein kann, eine hervorragende Sterilisationssicherheit gegeben, außerdem eine sehr hohe Stabilität des Versch lusselements.

Im Folgenden wird auf ein nicht zur Erfindung gehörendes Verfahren zur Herstellung eines Spritzensystems mit einem Verschlusselement 1 15 näher eingegangen. Zunächst wird hierzu Bezug genommen auf die Figuren 2 bis 4, die ein nicht zur Erfindung gehörendes Ausführungsbeispiel eines Verschlusselements zeigen.

Das Verschlusselement 1 wird in mehreren Verfahrensschritten hergestellt: Zunächst wird ein Stützkörper 3 in eine erste Montageposition im Inneren einer Verschlusshülse 5 eingebracht. In dieser Position wird der Stützkörper 3 durch eine Haltevorrichtung fixiert, die durch Rippen 21, 21' und 21" realisiert ist. Die Rippen halten den Stützkörper 3' in einem Abstand zur Innenfläche der Verschlusshülse 5, so dass durch den Zwischenraum ein Sterilisationsmedium in das Innere der Verschlusshülse einbringbar ist, so dass die späteren Anlageflächen zwischen dem Innenraum der Verschlusshülse 5 und dem Stützkörper 3' sterilisierbar sind.

Nach der Sterilisation wird der Stützkörper 3' in seine endgültige Position verlagert, die in Figur 4 dargestellt ist. In dieser Position wird er durch die Endbereiche der Rippen, die als Rastnasen dienen, fixiert.

Die Sterilisation der Anlagefläche zwischen zwei Teilen eines Verschlusselements 1 kann auch bei anderen Ausführungsbeispielen realisiert werden.

Vorzugsweise findet die Sterilisation in einem Autoklav statt, der auch als Schleuse dienen kann, durch die das Verschlusselement einer Fülleinrichtung zugeführt wird, in der eine Spritze mit einem Medikament und/oder Nährlösung oder dergleichen abfüllbar und durch das Verschlusselement verschließbar ist. Die Spritze kann gegebenenfalls auch noch nach dem Aufsetzen des Verschlusselements durch dessen Verlagerung weiter gefüllt werden.

## Patentansprüche

1. Verschlusselement (1),
- insbesondere zum Erschließen von Primärpackmitteln für Arzneimittel,
- das mindestens zwei Teile (3,5;41,45;41,55) aufweist,
- die zumindest bereichsweise aneinander liegen, sodass Anlageflächen gebildet werden,
wobei
- das erste Teil (3;45;55) eine höhere Festigkeit als des zweite Teil (5;41) aufweist,
- das erste Teil (3;45;55) als Stützkörper (3,3') ausgebildet ist, durch den Kräfte aufnehmbar sind, die in das zweite Teil (5;41) eingeleitet werden, und
- dampfdurchlässige Kanäle eines innerhalb Steritisationskörpers verlaufen,
**dadurch gekennzeichnet, dass**
- der Stützkörper (3,3') als der Sterilisationskörper ausgebildet ist, in dem die dampfdurchlässigen Kanäle zur Sterilisation der Anlageflächen vorgesehen sind.

2. Verschlusselement nach Anspruch 1, **dadurch gekennzeichnet, dass** die Kanäle als Mikrostrukturen, insbesondere durch Porosität des verwendeten Werkstoffs ausgebildet sind.

3. Verschlusselement nach einem der Ansprüche 1 oder 2, **dadurch gekennzeichnet, dass** der Sterilisationskörper als Klemmkörper (55) ausgebildet ist, der sicher an einem Primärpackmittel verankerbar ist.

4. Verschlusselement nach Anspruch 3, **dadurch gekennzeichnet, dass** der Klemmkörper (55) Anlageflächen für einen Verschlussstopfen (61) und eine Verschlusskappe (41) aufweist, wobei die Verschlusskappe (41) an dem Klemmkörper (55) und dieser seinerseits an dem Verschlussstopfen (61) anliegt.

5. Verschlusselement nach einem der vorhergehenden Ansprüche, **dadurch gekennzeichnet, dass** der Sterilisationskörper einen konischen Ansatz (53), insbesondere für eine Kanüle aufweist.

6. Verschlusselement nach einem der vorhergehenden Ansprüche, **dadurch gekennzeichnet, dass** der Sterilisationskörper eine Sicherungseinrichtung, insbesondere für eine Kanüle aufweist.

7. Verschlusselement nach Anspruch 4, **dadurch gekennzeichnet, dass** das Verschlusselement (1) einen durch die Verschlusskappe (41) verschließbaren Fluidkanal aufweist.

8. Verschlusselement nach Anspruch 1, **dadurch gekennzeichnet, dass** der Stützkörper (3,3') als Sicherheitskappe (45) ausgebildet ist.

9. Verschlusselement nach Anspruch 4, **gekennzeichnet durch** eine zusätzliche Sicherheitskappe (45), die die Verschlusskappe (41) übergreift.

10. Verschlusselement nach Anspruch 9, **dadurch gekennzeichnet, dass** die Sicherheitskappe (45) dampfdurchlässige Kanäle aufweist.

11. Verschlusselement nach Anspruch 1, **dadurch gekennzeichnet, dass** das zweite Teil (5;41) als Verschlusshülse (5) ausgebildet ist,

12. Verschlusselement nach Anspruch 11, **dadurch gekennzeichnet, dass** der Stützkörper (3,3') innerhalb der Verschlusshülse (5) angeordnet ist.

13. Verschlusselement nach einem der Ansprüche 11 oder 12, **dadurch gekennzeichnet, dass** die Verschlusshülse (5) eine Haltevorrichtung aufweist.

14. Verschlusselement nach einem der vorhergehenden Ansprüche 11 bis 13, **dadurch gekennzeichnet, dass** eine Rastvorrichtung vorgesehen ist, durch die die Teile (3,5;41,45;41,55) miteinander verrastbar sind.

15. Verschlusselement nach Anspruch 14, **dadurch gekennzeichnet, dass** die Rastvorrichtung durch eine Stufe (7,7'), die die Verschlusshülse (5) aufweist, gebildet wird.

## Claims

1. Closure element (1),
- particularly for closing primary packaging for pharmaceuticals,
- comprising at least two parts (3, 5; 41, 45; 41,55),
- which rest on each other at least area wise so that contact surfaces are formed,
wherein
- the first part (3; 45; 55) has a higher strength than the second part (5; 41),
- the first part (3; 45; 55) is designed as a support body (3, 3') by means of which forces can be absorbed which are introduced into the second part (5; 41), and
- vapor-permeable channels extend within a sterilization body,
**characterized in that**
- the support body (3, 3') is designed as the sterilization body in which the vapor-permeable channels are provided for the sterilization of the contact surfaces.

2. Closure element according to claim 1, **characterized in that** the channels are designed as microstructures, particularly due to the porosity of the material used.

3. Closure element according to any one of the claims 1 or 2, **characterized in that** the sterilization body is designed as a clamping body (55) which is securely anchorable on a primary packaging.

4. Closure element according to claim 3, **characterized in that** the clamping body (55) comprises contact surfaces for a closure plug (61) and a closure cap (41), wherein the closure cap (41) rests on the clamping body (55) and this, in turn, on the closure plug (61).

5. Closure element according to any one of the preceding claims, **characterized in that** the sterilization body comprises a conical extension (53), particularly for a cannula.

6. Closure element according to any one of the preceding claims, **characterized in that** the sterilization body comprises a safety device, particularly for a cannula.

7. Closure element according to claim 4, **characterized in that** the closure element (1) comprises a fluid channel closable by the closure cap (41).

8. Closure element according to claim 1, **characterized in that** the support body (3, 3') is designed as a safety cap (45).

9. Closure element according to claim 4, **characterized by** an additional safety cap (45) which overlaps the closure cap (41).

10. Closure element according to claim 9, **characterized in that** the safety cap (45) comprises vapor-permeable channels.

11. Closure element according to claim 1, **characterized in that** the second part (5; 41) is designed as a closure sleeve (5).

12. Closure element according to claim 11, **characterized in that** the support body (3, 3') is arranged within the closure sleeve (5).

13. Closure element according to any one of the claims 11 or 12, **characterized in that** the closure sleeve (5) comprises a holding device.

14. Closure element according to any one of the preceding claims 11 to 13, **characterized in that** a latching device is provided by means of which the parts (3, 5; 41, 45; 41, 55) can be latched with each other.

15. Closure element according to claim 14, **characterized in that** the latching device is formed by a step (7, 7') which the closure sleeve (5) comprises.

## Revendications

1. Elément de fermeture (1),
- notamment pour la fermeture de matériaux d'emballage primaire pour médicaments,
- qui présente au moins deux parties (3, 5 ; 41, 45 ; 41, 55),
- qui reposent l'une sur l'autre au moins par région de sorte que des surfaces d'appui sont formées,
- la première partie (3 ; 45 ; 55) présentant une solidité supérieure à la seconde partie (5 ; 41),
- la première partie (3 ; 45 ; 55) étant réalisée en tant que corps de support (3, 3') par lequel des forces qui sont introduites dans la seconde partie (5 ; 41) peuvent être absorbées, et
- des canaux perméables à la vapeur s'étendant au sein d'un corps de stérilisation,
**caractérisé en ce que**
- le corps de support (3, 3') est réalisé en tant que le corps de stérilisation dans lequel les canaux perméables à la vapeur sont prévus pour la stérilisation des surfaces d'appui.

2. Elément de fermeture selon la revendication 1, **caractérisé en ce que** les canaux sont réalisés en tant que microstructures, notamment par la porosité du matériau utilisé.

3. Elément de fermeture selon l'une quelconque des revendications 1 ou 2, **caractérisé en ce que** le corps de stérilisation est réalisé en tant que corps de serrage (55) qui peut être ancré de manière sûre sur un matériau d'emballage primaire.

4. Elément de fermeture selon la revendication 3, **caractérisé en ce que** le corps de serrage (55) présente des surfaces d'appui pour un bouchon de fermeture (61) et un capuchon (41), le capuchon (41) reposant sur le corps de serrage (55) et celui-ci reposant de son côté sur le bouchon de fermeture (61).

5. Elément de fermeture selon l'une quelconque des revendications précédentes, **caractérisé en ce que** le corps de stérilisation présente une rallonge conique (53), notamment pour une canule.

6. Elément de fermeture selon l'une quelconque des revendications précédentes, **caractérisé en ce que** le corps de stérilisation présente un dispositif de sécurité, notamment pour une canule.

7. Elément de fermeture selon la revendication 4, **caractérisé en ce que** l'élément de fermeture (1) présente un canal fluidique pouvant être fermé par le capuchon (41).

8. Elément de fermeture selon la revendication 1, **caractérisé en ce que** le corps de support (3, 3') est réalisé en tant que capuchon de sûreté (45).

9. Elément de fermeture selon la revendication 4, **caractérisé par** un capuchon de sûreté supplémentaire (45) qui chevauche le capuchon (41).

10. Elément de fermeture selon la revendication 9, **caractérisé en ce que** le capuchon de sûreté (45) présente des canaux perméables à la vapeur.

11. Elément de fermeture selon la revendication 1, **caractérisé en ce que** la seconde partie (5 ; 41) est réalisée en tant que manchon de fermeture (5).

12. Elément de fermeture selon la revendication 11, **caractérisé en ce que** le corps de support (3, 3') est disposé au sein du manchon de fermeture (5).

13. Elément de fermeture selon l'une quelconque des revendications 11 ou 12, **caractérisé en ce que** le manchon de fermeture (5) présente un dispositif de retenue.

14. Elément de fermeture selon l'une quelconque des revendications précédentes 11 à 13, **caractérisé en ce qu'**un dispositif d'encliquetage est prévu à travers lequel les parties (3, 5 ; 41, 45 ; 41, 55) peuvent être encliquetées ensemble.

15. Elément de fermeture selon la revendication 14, **caractérisé en ce que** le dispositif d'encliquetage est formé par un gradin (7, 7') qui présente le manchon de fermeture (5).
